# EUROPÄISCHE PATENTSCHRIFT

(11) **EP 1 066 239 B1**
(45) Veröffentlichungstag und Bekanntmachung des Hinweises auf die Patenterteilung: **28.05.2003**
(21) Anmeldenummer: 99913294.7
(22) Anmeldetag: 24.03.1999
(51) Int. Cl.: C07C 51/21, C07C 51/44, C07C 57/05, C07C 67/08, C07C 69/54

(54) **VERFAHREN ZUR HERSTELLUNG VON ACRYLSÄURE UND ACRYLSÄUREESTERN**
METHOD FOR PRODUCING ACRYLIC ACID AND ACRYLIC ACID ESTERS
PROCEDE DE PREPARATION D'ACIDE ACRYLIQUE ET D'ESTERS D'ACIDE ACRYLIQUE

(30) Priorität: 31.03.1998 DE 19814387
(43) Veröffentlichungstag der Anmeldung: 10.01.2001
(73) Patentinhaber: BASF AKTIENGESELLSCHAFT, 67056 Ludwigshafen (DE)
(72) Erfinder: NESTLER, Gerhard, A-1070 Wien (AU); SCHRÖDER, Jürgen, D-67071 Ludwigshafen (DE); MACHHAMMER, Otto, D-68163 Mannheim (DE)
(74) Vertreter: Isenbruck, Günter
(86) Internationale Anmeldenummer: EP9901995
(87) Internationale Veröffentlichungsnummer: WO99050219

(56) Entgegenhaltungen:
- EP-A- 0 009 545
- DE-A- 19 547 485
- DE-A- 19 740 253
- US-A- 3 555 082

## Beschreibung

Die vorliegende Erfindung betrifft ein Verfahren zur Herstellung von Acrylsäure, in dem ein bei der Gasphasenoxidation zur Herstellung von Acrylsäure anfallendes gasförmiges Reaktionsgemisch, das Acrylsäure enthält, mittels oligomerer Acrylsäure oder einem Gemisch aus Acrylsäure und oligomerer Acrylsäure abgekühlt wird, und ein Acrylsäure enthaltendes gasförmiges Gemisch erhalten wird. Ferner betrifft sie ein Verfahren zur Herstellung von Acrylsäureestern. Darüber hinaus betrifft sie ganz allgemein die Verwendung von oligomerer Acrylsäure bzw. eines Gemischs aus Acrylsäure und oligomerer Acrylsäure zum Abkühlen eines bei der Gasphasenoxidation zur Herstellung von Acrylsäure anfallenden gasförmigen Reaktionsgemischs.

Acrylsäure bildet aufgrund ihrer sehr reaktionsfähigen Doppelbindung sowie der Säurefunktion ein wertvolles Monomeres zur Herstellung von Polymerisaten, z.B. für als Klebstoffe geeignete wäßrige Polymerisatdispersionen.

Unter anderem ist Acrylsäure zugänglich durch Gasphasenoxidation von Propylen und/oder Acrolein mit Sauerstoff oder Sauerstoff enthaltenden Gasen in Gegenwart von Katalysatoren bei erhöhter Temperatur, infolge der hohen Reaktionswärme vorzugsweise unter Verdünnen der Reaktionspartner mit Inertgasen und/oder Wasserdampf.

Als Katalysatoren werden dabei in der Regel oxidische Mehrkomponentensysteme, z.B. auf Basis von Molybdän-, Chrom-, Vanadium- oder Telluroxiden, eingesetzt.

Bei diesem Verfahren wird jedoch nicht reine Acrylsäure, sondern ein Gasgemisch, das neben Acrylsäure als Nebenkomponenten im wesentlichen nicht umgesetztes Acrolein und/oder Propylen, Wasserdampf, Kohlenoxide, Stickstoff, Sauerstoff, Essigsäure, Formaldehyd, Benzaldehyd, Furfurale und Maleinsäureanhydrid enthält, erhalten, von welchem die Acrylsäure anschließend abgetrennt werden muß.

Die Isolierung der Acrylsäure aus dem gasförmigen Reaktionsgemisch erfolgt in der Regel durch Gegenstromabsorption z.B. mit einem hochsiedenden Lösungsmittel bzw. Lösungsmittelgemisch und mehreren anschließenden destillativen Aufarbeitungsschritten, wie dies z.B. in der DE-A 21 36 396 und der DE-A 43 08 087 beschrieben wird. Gemäß der EP-B 0 009 545, US 5 154 800, DE-A 34 29 391 sowie der DE-A 21 21 123 wird zunächst mit Wasser/wäßriger Acrylsäure im Gegenstrom absorbiert und anschließend extraktiv oder azeotrop destilliert.

Nachteilig bei diesen Verfahren ist es, daß sie in der Regel technisch und energetisch aufwendig sind und daß zur Absorption bzw. Extraktion ein zusätzliches organisches Lösungsmittel/Lösungsmittelgemisch notwendig ist, das in einem eigenen Destillationsschritt wieder abgetrennt und gegebenenfalls vor der Wiederverwendung gereinigt werden muß.

Ein weiterer Nachteil dieser Verfahren besteht darin, daß die bei der Propylenoxidation neben Acrylsäure anfallende Essigsäure (Gehalt: 0,5 bis 10 Gew.-% bezogen auf die Menge der Acrylsäure) in einer aufwendigen Destillationsstufe abgetrennt werden muß. Aufgrund der geringen Siedepunktsunterschiede und der hohen Polymerisationsneigung der Acrylsäure stellt dies eine schwierige Aufgabe dar, wie sich u.a. der US 3 844 903 entnehmen läßt.

Im Hinblick auf die bekannte Tatsache, daß Acrylverbindungen eine große Neigung zur Polymerisation besitzen, sind ganz allgemein Verfahren mit mehrstufiger destillativer Aufarbeitung nachteilig, da sie die Polymerisationstendenz der Acrylsäure noch verstärken.

Die Aufgabe der vorliegenden Erfindung besteht nunmehr darin, ein einfaches Verfahren zur Gewinnung von Acrylsäure zur Verfügung zu stellen, das zum einen kein zusätzliches Lösungs-/Absorptions- bzw. Extraktionsmittel benötigt und das darüber hinaus energetisch günstig durchführbar ist.

Demgemäß betrifft die vorliegende Erfindung ein Verfahren zur Herstellung von Acrylsäure, das folgende Stufe A umfaßt:
A: Abkühlen eines bei der Gasphasenoxidation zur Herstellung von Acrylsäure anfallenden gasförmigen Reaktionsgemischs, das Acrylsäure enthält, mittels oligomerer Acrylsäure oder eines Gemischs aus Acrylsäure und oligomerer Acrylsäure, wobei ein Acrylsäure enthaltendes, gasförmiges Gemisch und ein Quench-Sumpfprodukt, das oligomere Acrylsäure enthält, erhalten werden.

Der Begriff "gasförmiges Reaktionsgemisch, das Acrylsäure enthält" umfaßt erfindungsgemäß alle Reaktionsgemische, die bei der Gasphasenoxidation zur Herstellung von Acrylsäure anfallen.

Geht man von Propylen/Acrolein als Edukte zur Herstellung von Acrylsäure aus, handelt es sich um ein gasförmiges Reaktionsgemisch, das mit einer Temperatur von 200 bis 300°C aus der Gasphasenoxidation anfällt und ungefähr 1 bis 30 Gew.-% Acrylsäure enthält und als Nebenprodukte nicht umgesetztes Propylen (0,05 bis 1 Gew.-%), Acrolein (0,001 bis 2 Gew.-%), Propan (0,01 bis 2 Gew.-%), Wasserdampf (1 bis 30 Gew.-%), Kohlenoxide (0,05 bis 15 Gew.-%), Stickstoff (0 bis 90 Gew.-%), Sauerstoff (0,05 bis 10 Gew.-%), Essigsäure (0,05 bis 2 Gew.-%), Propionsäure (0,01 bis 2 Gew.-%), Aldehyde (0,05 bis 3 Gew.-%) und Maleinsäureanhydrid (0,01 bis 0,5 Gew.-%) umfaßt.

Dieses gasförmige Reaktionsgemisch wird mittels oligomerer Acrylsäure oder einem Gemisch aus Acrylsäure und oligomerer Acrylsäure in der Regel auf eine Temperatur von 100 bis 190°C, vorzugsweise 120 bis 80°C und insbesondere 130 bis 160°C abgekühlt, wobei ein wiederum gasförmiges, Acrylsäure enthaltendes Gemisch erhalten wird.

Als Abkühlvorrichtung können alle aus dem Stand der Technik für diesen Zweck bekannten Vorrichtungen eingesetzt werden, wobei vorzugsweise Venturi-Wäscher oder Sprühkühler (Quench) und insbesondere Sprühkühler verwendet werden.

Der erfindungsgemäß verwendete Begriff "oligomere Acrylsäure" umfaßt hier das durch Addition der Carboxylgruppe an die olefinische Doppelbindung durch Michael-Addition entstehende Produkt der folgenden allgemeinen Formel I:

CH₂=CHCO₂-(CH₂CH₂CO₂)ₙ-H n = 1 - 10

Die Bildung von oligomerer Acrylsäure tritt bei der Acrylsäureherstellung immer auf und wird von der Temperatur und der Verweildauer beeinflußt, läßt sich jedoch durch Inhibitoren nicht verhindern bzw. beeinflussen.

Das so erhaltene Acrylsäure enthaltende gasförmige Gemisch wird vorzugsweise in einer Stufe B aufgetrennt, wobei eine Roh-Acrylsäure, eine Leichtsiederfraktion und ein Sumpfprodukt, das oligomere Acrylsäure enthält, erhalten wird. Insbesondere wird die Auftrennung gemäß Stufe B in einer Destillationskolonne durchgeführt, und zwar weiter bevorzugt derart, daß die Acrylsäure über einen Seitenabzug einer Destillationskolonne erhalten wird.

Dabei wird im allgemeinen wie folgt vorgegangen:

Das Acrylsäure enthaltende gasförmige Gemisch aus Stufe A wird in den unteren Teil einer Destillationskolonne geleitet, in der die gasförmigen Bestandteile und die Leichtsieder, insbesondere Aldehyde, Essigsäure und Wasser, über den Kopf der Kolonne abgetrennt werden.

Die Acrylsäure wird als Roh-Acrylsäure im unteren Drittel der Destillationskolonne über einen Seitenabzug abgezogen.

Die Hochsieder, hauptsächlich oligomere Acrylsäure, fallen im Sumpf der Destillationskolonne an.

Die für das erfindungsgemäße Verfahren einsetzbaren Kolonnen unterliegen keiner besonderen Beschränkung. Grundsätzlich eignen sich alle Kolonnen mit trennwirksamen Einbauten.

Als Kolonneneinbauten kommen alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. Die Kolonne umfaßt wenigstens eine Kühlvorrichtung. Hierfür eignen sich alle Wärmeüberträger oder Wärmetauscher, bei denen die bei der Kondensation frei werdende Wärme indirekt (extern) abgeführt wird. Hierfür können alle gängigen Apparate eingesetzt werden, wobei Rohrbündelwärmetauscher, Plattenwärmetauscher und Luftkühler bevorzugt sind. Geeignete Kühlmedien sind bei einem Luftkühler entsprechend Luft und bei anderen Kühlvorrichtungen Kühlflüssigkeiten, insbesondere Wasser. Ist nur eine Kühlvorrichtung vorgesehen, so wird diese am Kopf der Kolonne eingebaut, in dem die Leichtsiederfraktion auskondensiert wird.

Der Fachmann kann in Abhängigkeit von der gewünschten Reinheit der kondensierten Fraktionen und damit der Komponenten die Anzahl der erforderlichen Kühlvorrichtungen leicht bestimmen, wobei die Reinheit der kondensierten Komponenten im wesentlichen durch die installierte Trennleistung der Kolonne, d.h. die Kolonnenhöhe, die Anzahl der Böden und die über das zu kondensierende gasförmige Gemisch aus Stufe A eingetragene Energie bestimmt wird. Zweckmäßigerweise werden bei Vorhandensein mehrerer Kühlvorrichtungen diese in verschiedenen Abschnitten der Kolonne eingebaut.

Z.B. können bei einem gasförmigen Gemisch aus Stufe A, das neben einem hohen Anteil nicht kondensierbarer Komponenten eine Schwersieder-, Mittelsieder- und Leichtsiederfraktion enthält, eine Kühlvorrichtung im unteren Abschnitt der Kolonne zur Auskondensation der Schwersiederfraktion und eine Kühlvorrichtung am Kopf der Kolonne zur Auskondensation der Leichtsiederfraktion vorgesehen sein. Die kondensierten Fraktionen werden an den jeweiligen Abschnitten in der Kolonne über Seitenabzüge abgeführt. In Abhängigkeit von der Anzahl der Komponenten in der Schwersieder-, Mittelsieder- und Leichtsiederfraktion können jeweils auch mehrere Seitenabzüge vorgesehen sein. Die über die Seitenabzüge abgezogenen Fraktionen können dann weiteren Reinigungsstufen unterzogen werden, z.B. destillativen oder extraktiven Trennverfahren oder einer Kristallisation, je nach gewünschter Reinheit der Komponenten.

In einer bevorzugten Ausgestaltung der Erfindung sind ein Schwersiederabzug, ein Leichtsiederabzug und 1 oder 2 Mittelsiederabzüge vorgesehen.

Der in der Kolonne vorliegende Druck hängt von der Menge an nicht kondensierbaren Komponenten ab und beträgt vorzugsweise 0,5 - 5 bar Absolutdruck, insbesondere 0,8 - 3 bar Absolutdruck.

Die Temperatur im Bereich der Auftrennvorrichtung, in dem die Leichtsieder, d.h. im wesentlichen typischerweise Aldehyde, Essigsäure und Wasser, abgetrennt werden, beträgt 25 bis 50°C, vorzugsweise 30 bis 40°C, die Temperatur im Bereich, in dem die Roh-Acrylsäure erhalten wird, beträgt 70 bis 110°C, vorzugsweise 80 bis 100°C, und die Sumpftemperatur wird bei 90 bis 140, insbesondere bei 115 bis 135°C gehalten wird.

Die genauen Betriebsbedingungen für die Kolonne, wie Temperatur- und Druckführung, Schaltung und Anordnung der Kühlvorrichtung(en), Anordnung der Seitenabzüge zum Abziehen der gewünschten Fraktionen, Wahl der Kolonnenhöhe und des Kolonnendurchmessers, Anzahl und Abstand der trennwirksamen Einbauten/Böden in der Kolonne sowie die Art der trennwirksamen Kolonneneinbauten, können vom Fachmann im Rahmen fachüblicher Versuche in Abhängigkeit von der Trennaufgabe ermittelt werden.

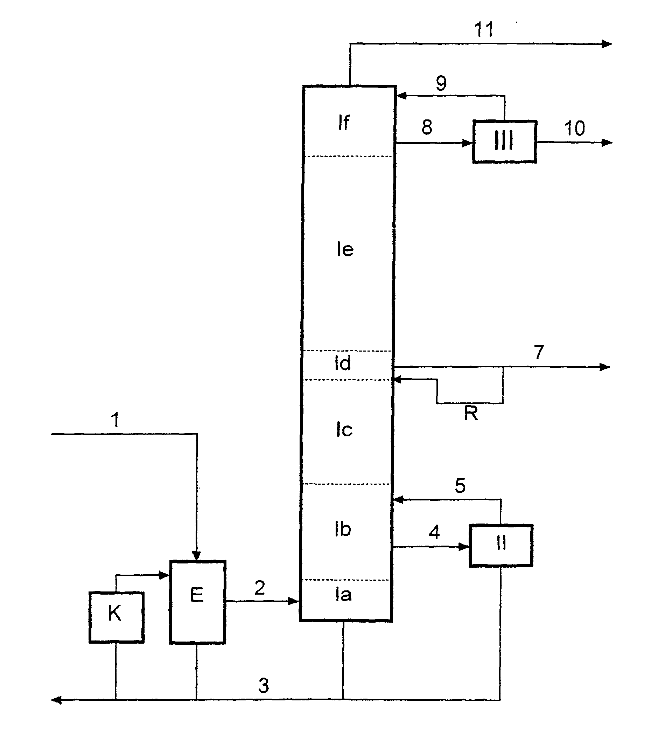

Vorteilhafterweise wird das Verfahren bei Vorhandensein einer Schwersiederfraktion, einer Mittelsiederfraktion, einer Leichtsiederfraktion und nicht kondensierbarer Komponente(n) in dem Acrylsäure enthaltenden, gasförmigen Gemisch (gasförmiges Gemisch) so durchgeführt, wie es in der Figur gezeigt ist und wie es im folgenden beschrieben ist, wobei sich die Kolonne in verschiedene Abschnitte untergliedern läßt, in denen unterschiedliche verfahrenstechnische Aufgaben gelöst werden.

Die Bezugsziffern in der Figur bezeichnen hierbei die einzelnen Abschnitte in der Kolonne (I.a bis I.f) bzw. separate Abschnitte/Apparate vor der Kolonne (E), Zuund Ableitungen (1 - 11) sowie die Kühlkreise II und III.

### E. Quench:

### Abkühlung des gasförmigen Gemischs

In der Einrichtung E wird das gasförmige Gemisch eingeleitet und abgekühlt. Dies kann z.B. über direkte Kühlung mit im nächsten Abschnitt der Kolonne kondensierter Schwersiederfraktion, die oligomere Acrylsäure enthält, als Kühlmedium erfolgen. Dabei wird das gasförmige Gemisch aus Leitung 1 in einem Quench E abgekühlt und über Leitung 2 dem Sumpfbereich I.a der Kolonne zugeführt. Über Leitung 3 wird die kondensierte Schwersiederfraktion zur Kühlung des gasförmigen Gemischs in den Quench zurückgeführt. Dabei kann die zur Kühlung zurückgeführte oligomere Acrylsäure (Schwersieder) in einer Kühlvorrichtung (K) abgekühlt, z.B. auf 80 bis 150°C abgekühlt werden. Ein Teil des Stromes, üblicherweise 0,1 bis 10 Gew.-%, bezogen auf 100 Gew.-% Acrylsäure, wird aus dem Prozeß ausgeschleust.

### I.b Kühlkreis II:

### Kondensation der Schwersiederfraktion

Im Kolonnenabschnitt I.b wird die Kondensationswärme extern über Kühlkreis II mittels eines Wärmetauschers mit z.B. Wasser als Kühlmedium abgeführt, indem kondensierte Schwersiederfraktion über Leitung 4 aus der Kolonne abgeführt wird, gekühlt wird und ein Teil der gekühlten, kondensierten Schwersiederfraktion über Leitung 5 der Kolonne rückgeführt wird, während der andere Teil entsprechend dem Stand im Quench standgeregelt über Leitung 3 in den Quench E zurückgeführt wird. Die rückgeführte, kondensierte Schwersiederfraktion wird im Gegenstrom zum aufsteigenden gasförmigen Gemisch geführt.

### I.c Kühlkreis II → Seitenabzug:

### Schwersiederanreichung

Im Kolonnenabschnitt I.c zwischen Kolonnenabschnitt I.b (Kühlkreis II) und I.d (Seitenabzug) erfolgt zum Kühlkreis II hin eine destillative Anreicherung und Auskondensation der Schwersiederfraktion aus dem im Gegenstrom nach oben geführten gasförmigen Gemisch.

### I.d Seitenabzug:

### Abziehen der Mittelsiederfraktion

Über Seitenabzug 7 im Kolonnenabschnitt I.d. wird die gewünschte Zielkomponente Acrylsäure als Roh-Acrylsäure z.B. über einen Fangboden flüssig abgeführt und teilweise als Rücklauf (R), gegebenenfalls über einen Wärmetauscher unterhalb des Seitenabzugs 7 in die Kolonne zurückgeführt.

### I.e Seitenabzug → Kühlkreis III:

### Mittelsiederanreicherung

Im Kolonnenabschnitt I.e zwischen Kolonnenabschnitt I.d (Seitenabzug 7) und I.f (Kühlkreis III) erfolgt die destillative Anreicherung der Mittelsiederfraktion des gasförmigen Gemischs aus dem nach oben geführten gasförmigen Gemisch, wobei die Mittelsiederfraktion zum Seitenabzug (Bereich I.d) hin angereichert wird.

### I.f Kühlkreis III:

### Kondensation der Leichtsiederfraktion

Im Kolonnenabschnitt I.f des externen Kühlkreises III erfolgt die Kondensation der Leichtsiederfraktion aus dem im Gegenstrom nach oben geführten gasförmigen Gemisch. Analog zu Kühlkreis II wird die Kondensationswärme extern über Kühlkreis III mittels eines Wärmetauschers mit z.B. Wasser als Kühlmedium abgeführt, indem kondensierte Leichtsiederfraktion über Leitung 8 abgezogen wird, gekühlt wird und ein Teil der gekühlten, kondensierten Leichtsiederfraktion über Leitung 9 der Kolonne rückgeführt wird, während der andere Teil über Leitung 10 ausgeschleust wird. Die nicht kondensierten Gase werden vom Kopf der Kolonne über Leitung 11 abgezogen, wobei gegebenenfalls der Gasstrom noch überhitzt werden kann, um eine weitere Kondensation im Brüdenrohr zu vermeiden.

Das Gas wird vorzugsweise als Kreisgas über Leitung 11 in die Acrylsäure-Herstellung zurückgefahren.

Weitere Details bezüglich dieser Verfahrensweise sind der DE-A 197 40 253 zu entnehmen, deren Inhalt durch Bezugnahme vollumfänglich in den Kontext der vorliegenden Anmeldung aufgenommen wird.

Während des Auftrennens wird zur Stabilisierung ein Polymerisationsinhibitor, wie z.B. Phenothiazin, eine phenolische Verbindung, eine N-O-Verbindung oder ein Gemisch aus zwei oder mehr davon, vorzugsweise Phenothiazin oder Hydrochinon, ein Gemisch aus Phenothiazin und Hydrochinon, Hydrochinonmonomethylether, p-Nitrosophenol, Nitrosodiethylanilin oder Tetramethylpiperidin-1-oxylen, wie sie in der DE-A-16 18 141 beschrieben sind, zugegeben.

Die nach der Auftrennung erhaltenen Leichtsieder werden nach dem Ausschleusen aus der Auftrennvorrichtung ganz oder teilweise, gegebenenfalls unter Zugabe eines Polymerisationsinhibitors als Rücklauf wieder in den oberen Teil der Auftrennvorrichtung zurückgeführt, um dort die Kondensation der im Acrylsäure enthaltenen gasförmigen Gemisch enthaltenen Leichtsieder zu erleichtern.

Die als Mittelsieder vorzugsweise über einen Seitenabzug erhaltene Roh-Acrylsäure wird der Kristallisation bzw. Destillation nach einem aus dem Stand der Technik bekannten Verfahren unterworfen, wobei eine Rein-Acrylsäure erhalten wird. Dabei wird Mutterlauge aus der Kristallisation ganz oder teilweise und/oder ggf. ein Teil der Roh-Acrylsäure unterhalb des Seitenabzugs der Kolonne zugeführt.

Darüber hinaus kann die erfindungsgemäß erhaltene Roh-Acrylsäure auch der Veresterung nach einem Verfahren gemäß des Standes der Technik, wie dies beispielsweise in der DE-A-195 47 485 und dem dort zitierten Stand der Technik beschrieben ist, zugeführt werden.

Demgemäß betrifft die vorliegende Erfindung auch ein Verfahren zur Herstellung eines Acrylsäureesters oder eines Gemischs aus zwei oder mehr davon, das zusätzlich zu den oben definierten Stufen A und B eine weitere Stufe C umfaßt:

### C: Veresterung der in Stufe B erhaltenen Roh-Acrylsäure mittels einem oder mehrerer Alkanole.

Für weitere Details bezüglich der Veresterung von Acrylsäure wird auf die oben erwähnte DE-A 195 47 485 und den darin zitierten Stand der Technik verwiesen, die vollumfänglich in den Kontext der vorliegenden Anmeldung durch Bezugnahme einbezogen wird.

Der in Stufe A anfallende Quench-Sumpf besteht hauptsächlich, d.h. zu 60 bis 100 Gew.-%, aus oligomerer Acrylsäure. Diese oligomere Acrylsäure kann im Rahmen des vorliegenden Verfahrens wie folgt weiterverwendet werden:
1. Die in Stufe B anfallende Schwersiederfraktion wird teilweise - entsprechend dem Stand im Quench - diesem über eine Abkühlvorrichtung, vorzugsweise einen herkömmlichen Wärmeaustauscher, zugeführt, auf eine Temperatur im Bereich von im allgemeinen 40 bis 100°C, vorzugsweise 80 bis 100°C abgekühlt und zum Abkühlen des bei der Gasphasenoxidation zur Herstellung von Acrylsäure anfallenden gasförmigen Reaktionsgemischs, das Acrylsäure enthält, in Stufe A zurückgeführt.
2. Die aus dem Quench ausgeschleuste oligomere Acrylsäure wird thermisch, vorzugsweise in Gegenwart eines Katalysators, wie sie beispielsweise in Ullmanns Encyklopädie der techn. Chemie, 4. Aufl., Bd. 7, S. 83 beschrieben sind, vorzugsweise eine Mineralsäure oder eine von Acrylsäure verschiedene organische Säure, wie z.B. Alkyl- oder Arylsulfonsäure, insbesondere Schwefel-, Phosphor-, Methansulfon- oder p-Toluolsulfonsäure, wieder in Acrylsäure gespalten. Die dabei als Destillat anfallenden Spaltprodukte, also hauptsächlich Acrylsäure und Diacrylsäure, können dann entweder mit der in Stufe B erhaltenen Roh-Acrylsäure vereinigt und weiter aufgearbeitet bzw. weiterverarbeitet werden oder in die Stufe A zum Abkühlen des bei der Gasphasenoxidation anfallenden gasförmigen Reaktionsgemischs zurückgeführt werden. Bei der Abkühlung des gasförmigen Reaktionsgemischs verdampft diese Acrylsäure und wird auf diese Weise wieder zurückgewonnen.
3. Eine weitere vorteilhafte Ausführungsform der vorliegenden Erfindung besteht darin, daß die anfallende oligomere Acrylsäure gemeinsam mit den bei der Acrylsäureesterherstellung üblicherweise anfallenden Oxyestern gespalten wird, wie dies in der DE-A 195 47 485 beschrieben wird. Demgemäß wird das bei der Veresterung anfallende Sumpfprodukt, das die Oxyester enthält, zunächst abgetrennt und anschließend
   - diesem Sumpfprodukt unmittelbar oligomere Acrylsäure zugesetzt; die im Sumpfprodukt enthaltenen Oxyester werden dann in Anwesenheit von von oligomerer Acrylsäure verschiedenen Säurekatalysatoren durch Einwirkung erhöhter Temperatur gespalten,
      oder
   - die Oxyester zunächst aus dem Sumpfprodukt der Acrylsäureesterherstellung destillativ abgetrennt, das Destillat mit oligomerer Acrylsäure versetzt und in Anwesenheit von von oligomerer Acrylsäure verschiedenen Säurekatalysatoren durch Einwirkung erhöhter Temperatur gespalten werden.

Dabei beträgt der Anteil der oligomeren Acrylsäure am zu spaltenden Gemisch 10 bis 50 Gew.-%, vorzugsweise 10 bis 40 Gew.-%.

Vorzugsweise werden dem zu spaltenden Gemisch zusätzlich zu dem gegebenenfalls bereits enthaltenen, von oligomerer Acrylsäure verschiedenen, sauren Veresterungskatalysator weitere Säuren aus der Gruppe umfassend Mineralsäuren, wie z.B. Schwefel- oder Phosphorsäure, und von oligomerer Acrylsäure verschiedenen organischen Säuren, wie z.B. Alkyl- oder Arylsulfonsäuren, insbesondere Methansulfonsäure oder p-Toluolsulfonsäure, zugesetzt. Die insgesamt enthaltene, von oligomerer Acrylsäure verschiedene Säuremenge kann dabei 1 bis 20 Gew.-%, vorzugsweise 5 bis 15 Gew.-%, bezogen auf die Menge des zu spaltenden Produkts, betragen. Die bei dieser Spaltung erhaltene Mischung, die hauptsächlich den Acrylsäureester, Alkanol und Acrylsäure enthält, wird entweder der Veresterung oder der Aufarbeitung des Roh-Acrylsäureesters zugeführt.

Weitere Details bezüglich dieser Spaltung von oligomerer Acrylsäure und dem Sumpfprodukt der Acrylsäureester-Herstellung lassen sich der oben erwähnten DE-A 195 47 485 entnehmen, die vollumfänglich in den Kontext der vorliegenden Anmeldung einbezogen wird.

Wie sich aus obigem ergibt, betrifft somit die vorliegende Erfindung auch ein Verfahren zur Herstellung von Acrylsäure, bzw. eines Acrylsäureesters, wobei ein Teil des in Stufe A erhaltenen Quench-Sumpfprodukts oder des in Stufe B erhaltenen Sumpfprodukts oder ein Gemisch davon, die jeweils oligomere Acrylsäure enthalten, in Gegenwart eines Katalysators erwärmt werden, wobei eine Acrylsäure enthaltende Mischung erhalten wird und ein Verfahren zur Herstellung von Acrylsäure, bzw. eines Acrylsäureesters, wobei ein Teil des in Stufe A erhaltenen Quench-Sumpfprodukts, das oligomere Acrylsäure enthält, und bei der Veresterung gemäß Stufe C anfallende hochsiedende Nebenprodukte ganz oder teilweise in Gegenwart eines Katalysators erwärmt werden, wobei eine Acrylsäure und Acrylsäureester enthaltende Mischung erhalten wird.

Die Acrylsäure enthaltende Mischung kann ganz oder teilweise zur Abkühlung gemäß Stufe A verwendet werden. Ferner können die Acrylsäure enthaltende Mischung und die Acrylsäure und Acrylsäureester enthaltende Mischung ganz oder teilweise, einzeln oder vereinigt, in die Veresterung zurückgefahren werden.

Das erfindungsgemäße Verfahren kann kontinuierlich oder aber diskontinuierlich durchgeführt werden, wobei die kontinuierliche Durchführung bevorzugt ist.

Das erfindungsgemäße Verfahren weist die folgenden Vorteile auf:
1. Es wird kein fremdes Absorptions- oder Extraktionsmittel benötigt. Es werden die bei der Acrylsäureherstellung immer anfallenden oligomeren Acrylsäuren an sich zur Abkühlung des bei der Gasphasenoxidation zur Herstellung von Acrylsäure anfallenden gasförmigen Reaktionsgemischs, das Acrylsäure enthält, eingesetzt.
2. Das Verfahren ist technisch vergleichsweise einfach, da nur eine Auftrennvorrichtung benötigt wird. Bedingt durch die Tatsache, daß das bei der Gasphasenoxidation erhaltene Reaktionsgemisch lediglich abgekühlt und in ein weiteres gasförmiges Gemisch überführt wird, ist das Verfahren darüber hinaus energetisch relativ günstig durchzuführen.
3. Die oligomere Acrylsäure kann wieder in Wertprodukt, d.h. Acrylsäure, überführt werden.

Die erfindungsgemäß gewonnene Roh-Acrylsäure kann durch Kristallisation oder Destillation direkt zu Rein-Acrylsäure verarbeitet oder mit Alkanolen verestert werden.

In ihrer allgemeinsten Form betrifft die vorliegende Erfindung auch die Verwendung von oligomerer Acrylsäure oder eines Gemischs aus Acrylsäure und oligomerer Acrylsäure zum Abkühlen eines bei der Gasphasenoxidation zur Herstellung von Acrylsäure anfallenden gasförmigen Reaktionsgemischs, das Acrylsäure enthält.

Die vorliegende Erfindung soll nunmehr noch anhand eines Beispiels erläutert werden.

### Beispiel

Durch zweistufige katalytische Oxidation von Propylen mit molekularem Sauerstoff wurde auf übliche Weise ein gasförmiges Reaktionsgemisch mit folgender Zusammensetzung erhalten:
9,84 Gew.-% Acrylsäure,
0,4 Gew.-% Essigsäure,
4,42 Gew.-% Wasser,
0,11 Gew.-% Acrolein,
0,21 Gew.-% Formaldehyd,
0,07 Gew.-% Maleinsäureanhydrid, sowie
Propionsäure, Furfural, Propan, Propen, Stickstoff, Sauerstoff und Kohlenoxide.

Dieses gasförmige Reaktionsgemisch wurde in einem Sprühkühler (Quench) durch Eindüsen von oligomerer Acrylsäure (800 l/h) auf 140°C abgekühlt. Die oligomere Acrylsäure wurde dabei über einen Wärmeaustauscher im Kreis gefahren und eine Temperatur von 95°C eingestellt.

Überflüssige oligomere Acrylsäure wurde in einer Menge von 3 g/h standgeregelt ausgeschleust.

Das abgekühlte, gasförmige Gemisch, das Acrylsäure enthielt, wurde über einen Tropfenabscheider (Zyklon) in den unteren Teil einer Destillationskolonne geleitet, die mit 60 Dual-Flow-Böden, einem Seitenabzug zwischen dem 14. und 15. Boden und einem Sprühkondensator am Kopf der Kolonne ausgerüstet war. Die Temperatur am Kopf der Destillationskolonne betrug 34°C, die Sumpftemperatur der Destillationskolonne betrug 100°C.

Das im Sprühkondensator anfallende Destillat, das hauptsächlich aus Wasser und Essigsäure bestand, wurde nach Ausschleusung von 20 % desselben und Zugabe von 2.000 ppm Hydrochinon als Rücklauf wieder auf den obersten Kolonnenboden aufgebracht. Durch den Seitenabzug wurden 350 g/h Roh-Acrylsäure aus der Destillationskolonne flüssig entnommen. Diese Roh-Acrylsäure enthielt 97,3 Gew.-% Acrylsäure, 0,9 Gew.-% Essigsäure, 0,05 Gew.-% Propionsäure, 0,01 Gew.-% Acrolein, 0,03 Gew.-% Furfural und 1,5 Gew.-% Wasser.

Die im Sumpf der Destillationskolonne anfallende Schwersiederfraktion wurde teilweise - entsprechend dem Stand im Quench - diesem zugeführt. Der übrige Teil der Schwersiederfraktion wurde über einen Wärmeaustauscher geleitet und auf den fünften Kolonnenboden zugefahren, wobei eine Temperatur von 100°C eingestellt wurde.

Das aus dem Quench-Sumpf ausgeschleuste Sumpfprodukt mit einem Anteil an oligomerer Acrylsäure von ca. 80 Gew.-% wurde in einem beheizbaren 2 l-Rührreaktor in Gegenwart von 1 Gew.-% Schwefelsäure 3 Stunden lang auf 150°C erhitzt, wobei die entstehenden Spaltprodukte gasförmig über einen Spritzschutz kontinuierlich abgezogen und kondensiert wurden. Das Destillat (91 Gew.-% der Einsatzmenge) bestand hauptsächlich aus Acrylsäure und Diacrylsäure und wurde mit 500 ppm Phenothiazin stabilisiert und ebenfalls dem Kühlkreislauf zur Abkühlung des gasförmigen Reaktionsgemischs zugeführt.

## Patentansprüche

1. Verfahren zur Herstellung von Acrylsäure, das folgende Stufe A umfaßt:
A: Abkühlen eines bei der Gasphasenoxidation zur Herstellung von Acrylsäure anfallenden gasförmigen Reaktionsgemischs, das Acrylsäure enthält, mittels oligomerer Acrylsäure oder eines Gemischs aus Acrylsäure und oligomerer Acrylsäure, wobei ein Acrylsäure enthaltendes, gasförmiges Gemisch und ein Quench-Sumpfprodukt, das oligomere Acrylsäure enthält, erhalten werden.

2. Verfahren nach Anspruch 1, **dadurch gekennzeichnet, daß** in Stufe A das gasförmige Reaktionsgemisch, das Acrylsäure enthält, auf eine Temperatur von 120 bis 180°C abgekühlt wird.

3. Verfahren nach Anspruch 1 oder 2, **dadurch gekennzeichnet, daß** in Stufe A das gasförmige Reaktionsgemisch, das Acrylsäure enthält, in einem Sprühkühler abgekühlt wird.

4. Verfahren nach einem der Ansprüche 1 bis 3, das folgende weitere Stufe B umfaßt:
B: Auftrennung des Acrylsäure enthaltenden, gasförmigen Gemischs, wobei eine Leichtsiederfraktion, eine Roh-Acrylsäure und ein Sumpfprodukt, das oligomere Acrylsäure enthält, erhalten wird.

5. Verfahren nach Anspruch 4, **dadurch gekennzeichnet, daß** die Auftrennung gemäß Stufe B in einer Destillationskolonne durchgerührt wird und Acrylsäure über einen Seitenabzug der Destillationskolonne erhalten wird.

6. Verfahren nach einem der Ansprüche 1 bis 5, **dadurch gekennzeichnet, daß** die oligomere Acrylsäure vollständig oder teilweise in die Abkühlung gemäß Stufe A zurückgeführt wird.

7. Verfahren nach einem der Ansprüche 1 bis 6, **dadurch gekennzeichnet, daß** die in Stufe B erhaltene Roh-Acrylsäure, vorzugsweise durch Kristallisation, in Rein-Acrylsäure überführt wird.

8. Verfahren zur Herstellung eines Acrylsäureesters oder eines Gemischs aus zwei oder mehr davon, **dadurch gekennzeichnet, daß** es eine Stufe A, wie in einem der Ansprüche 1 bis 3 definiert, eine Stufe B, wie in Anspruch 4 oder 5 definiert, sowie eine weitere Stufe C umfaßt:
C: Veresterung der in Stufe B erhaltenen Roh-Acrylsäure mittels einem oder mehreren Alkanolen.

9. Verfahren nach einem der Ansprüche 1 bis 8, **dadurch gekennzeichnet, daß** ein Teil des in Stufe A erhaltenen Quench-Sumpfprodukts oder des in Stufe B erhaltenen Sumpfprodukts oder ein Gemisch davon, die jeweils oligomere Acrylsäure enthalten, in Gegenwart eines Katalysators erwärmt werden, wobei eine Acrylsäure enthaltende Mischung erhalten wird.

10. Verfahren nach einem der Ansprüche 1 bis 8, **dadurch gekennzeichnet, daß** ein Teil des in Stufe A erhaltenen Quench-Sumpfprodukts, das oligomere Acrylsäure enthält, und bei der Veresterung gemäß Stufe C anfallende hochsiedende Nebenprodukte ganz oder teilweise in Gegenwart eines Katalysators erwärmt werden, wobei eine Acrylsäure und Acrylsäureester enthaltende Mischung erhalten wird.

11. Verfahren nach Anspruch 9, **dadurch gekennzeichnet, daß** die Acrylsäure enthaltende Mischung zur Abkühlung gemäß Stufe A verwendet wird.

12. Verwendung von oligomerer Acrylsäure oder eines Gemischs aus Acrylsäure und oligomerer Acrylsäure zum Abkühlen eines bei der Gasphasenoxidation zur Herstellung von Acrylsäure anfallenden gasförmigen Reaktionsgemischs, das Acrylsäure enthält.

## Claims

1. A process for preparing acrylic acid comprising the following stage A:
A: cooling a gaseous reaction mixture which comprises acrylic acid and is obtained in the gas-phase oxidation to prepare acrylic acid, using oligomeric acrylic acid or a mixture of acrylic acid and oligomeric acrylic acid, to give a gaseous mixture comprising acrylic acid and a quench bottom product which comprises oligomeric acrylic acid.

2. A process as claimed in claim 1, wherein the gaseous reaction mixture comprising acrylic acid is cooled in stage A to a temperature from 120 to 180°C.

3. A process as claimed in claim 1 or 2, wherein the gaseous reaction mixture comprising acrylic acid is cooled in stage A in a spray cooler.

4. A process as claimed in any one of claims 1 to 3, which comprises the following further stage B:
B: separating the gaseous mixture comprising acrylic acid, to give a low-boiling fraction, a crude acrylic acid, and a bottom product, which comprises oligomeric acrylic acid.

5. A process as claimed in claim 4, wherein the separation of stage B is carried out in a distillation column and acrylic acid is obtained via a sidestream takeoff of the distillation column.

6. A process as claimed in any one of claims 1 to 5, wherein some or all of the oligomeric acrylic acid is passed back into the cooling operation of stage A.

7. A process as claimed in any one of claims 1 to 6, wherein the crude acrylic acid obtained in stage B is converted into pure acrylic acid, preferably by crystallization.

8. A process for preparing an acrylate or a mixture of two or more thereof, which comprises a stage A as defined in any one of claims 1 to 3, a stage B as defined in claim 4 or 5, and a further stage C:
C: esterifying the crude acrylic acid obtained in stage B by means of one or more alkanols.

9. A process as claimed in any one of claims 1 to 8, wherein a portion of the quench bottom product obtained in stage A or of the bottom product obtained in stage B, or a mixture thereof, each of which comprises oligomeric acrylic acid, is heated in the presence of a catalyst, to give a mixture comprising acrylic acid.

10. A process as claimed in any one of claims 1 to 8, wherein a portion of the quench bottom product obtained in stage A, which comprises oligomeric acrylic acid, and high-boiling byproducts obtained in the esterification of stage C are heated in whole or in part in the presence of a catalyst, to give a mixture comprising acrylic acid and acrylate.

11. A process as claimed in claim 9, wherein the mixture comprising acrylic acid is used for cooling in stage A.

12. The use of oligomeric acrylic acid or of a mixture of acrylic acid and oligomeric acrylic acid to cool a gaseous reaction mixture comprising acrylic acid and obtained in the gas-phase oxidation to prepare acrylic acid.

## Revendications

1. Procédé de préparation d'acide acrylique, comprenant l'étape A suivante:
A: Refroidissement d'un mélange réactionnel provenant de l'oxydation en phase gazeuse pour la préparation d'acide acrylique, contenant de l'acide acrylique, au moyen d'acide acrylique oligomère ou d'un mélange d'acide acrylique et d'acide acrylique oligomère, avec obtention d'un mélange gazeux contenant de l'acide acrylique et d'un produit de fond de cuve /quench; qui contient de l'acide acrylique oligomère.

2. Procédé selon la revendication 1, **caractérisé en ce que**, dans l'étape A, le mélange réactionnel gazeux, qui contient de l'acide acrylique, est refroidi à une température de 120 à 180°C.

3. Procédé selon la revendication 1 ou 2, **caractérisé en ce que**, dans l'étape A, le mélange réactionnel gazeux, qui contient de l'acide acrylique, est refroidi dans un refroidisseur à pulvérisation.

4. Procédé selon l'une des revendications 1 à 3, comprenant en outre l'étape B suivante:
B: Séparation du mélange gazeux contenant de l'acide acrylique, avec obtention d'une fraction à bas point d'ébullition, d'un acide acrylique brut et d'un produit de fond de cuve, qui contient de l'acide acrylique oligomère.

5. Procédé selon la revendication 4, **caractérisé en ce que** la séparation selon l'étape B est entreprise dans une colonne de distillation et que l'on obtient de l'acide acrylique via un soutirage latéral de la colonne de distillation.

6. Procédé selon l'une des revendications 1 à 5, **caractérisé en ce que** l'acide acrylique oligomère est recyclé en tout ou en partie dans le refroidissement selon l'étape A.

7. Procédé selon l'une des revendications 1 à 6, **caractérisé en ce que** l'acide acrylique brut obtenu à l'étape B est converti en acide acrylique pur, de préférence par cristallisation.

8. Procédé de préparation d'un ester d'acide acrylique ou d'un mélange de deux ou plus de deux d'entre eux, **caractérisé en ce qu'**il comporte une étape A, telle que définie dans l'une des revendications 1 à 3, une étape B, telle que définie dans la revendication 4 ou 5, ainsi qu'une autre étape C:
C: Estérification de l'acide acrylique brut obtenu à l'étape B au moyen d'un ou plusieurs alcanols.

9. Procédé selon l'une des revendications 1 à 8, **caractérisé en ce qu'**une partie du produit de fond de cuve / quench obtenu à l'étape A ou du produit de fond de cuve obtenu à l'étape B, ou leur mélange, contenant à chaque fois de l'acide acrylique oligomère, est chauffée en présence d'un catalyseur, avec obtention d'un mélange contenant de l'acide acrylique.

10. Procédé selon l'une des revendications 1 à 8, **caractérisé en ce qu'**une partie du produit de fond de cuve / quench obtenu à l'étape A, contenant de l'acide acrylique oligomère et des sous-produits à haut point d'ébullition provenant de l'estérification selon l'étape C est chauffée en tout ou en partie en présence d'un catalyseur, avec obtention d'un mélange contenant de l'acide acrylique et des esters acryliques.

11. Procédé selon la revendication 9, **caractérisé en ce que** le mélange contenant de l'acide acrylique est utilisé pour le refroidissement selon l'étape A.

12. Utilisation d'acide acrylique oligomère ou d'un mélange d'acide acrylique et d'acide acrylique oligomère pour le refroidissement d'un mélange réactionnel gazeux provenant de l'oxydation en phase gazeuse pour la préparation d'acide acrylique, et contenant de l'acide acrylique.
